# EUROPÄISCHE PATENTSCHRIFT

(11) **EP 0 686 150 B1**
(45) Veröffentlichungstag und Bekanntmachung des Hinweises auf die Patenterteilung: **20.05.1998**
(21) Anmeldenummer: 94910321.2
(22) Anmeldetag: 24.02.1994
(51) Int. Cl.: C07D 239/02, C07D 405/04, C07H 21/00, C07H 19/06, C07H 19/10, C07F 9/6506, A61K 31/70

(54) **Verfahren zur Herstellung von C-Nukleosiden und C-Nukleosid-Analoga, neue C-Nukleoside und C-Nukleosid-Analoga, sowie ihre Verwendung**
Process for the preparation of C-nucleosides and C-nucleoside analogues, new C-nucleosides and C-nucleoside analogues and their use
Procédé pour la préparation des C-nucléosides et des analogues, nouveaux C-nucléosides et des analogues et leur utilisation

(30) Priorität: 26.02.1993 DE 4306859; 15.06.1993 DE 4320570
(43) Veröffentlichungstag der Anmeldung: 13.12.1995
(73) Patentinhaber: MAX-DELBRÜCK-CENTRUM FÜR MOLEKULARE MEDIZIN, D-13125 Berlin (DE)
(72) Erfinder: BÄRWOLFF, Dieter, D-13125 Berlin (DE)
(74) Vertreter: Baumbach, Friedrich, Dr.
(86) Internationale Anmeldenummer: DE9400205
(87) Internationale Veröffentlichungsnummer: WO9419327

(56) Entgegenhaltungen:
- EP-A- 0 103 464
- EP-A- 0 398 231
- JOURNAL OF ORGANIC CHEMISTRY. Bd. 43, Nr. 14 , 7. Juli 1978 , EASTON US Seiten 2925 - 2927 J.A.SECRIST III 'Homo-C-nucleosides. The Synthesis of Certain 6-Substituted 4-Pyrimidinones'
- JOURNAL OF ORGANIC CHEMISTRY. Bd. 47, Nr. 26 , 17. Dezember 1982 , EASTON US Seiten 5115 - 5120 T.L.CUPPS ET AL. 'Use of Allyltrimethylsilane in the Formation of Potential C-Nucleoside Precursors.'
- JOURNAL OF ORGANIC CHEMISTRY. Bd. 51, Nr. 7 , 4. April 1986 , EASTON US Seiten 1058 - 1064 T.L.CUPPS ET AL. 'A Novel Three-Step Synthesis of a Pyrrolo(3,2-d)pyrimidine C-Nucleoside.'
- LIEBIGS ANNALEN DER CHEMIE Nr. 6 , 12. Juni 1986 , WEINHEIM DE Seiten 957 - 966 G.RENZ ET AL. 'Synthesen von 2,4-substituierten Homo-C-pyrimidin-nucleosiden.'
- JOURNAL OF THE CHEMICAL SOCIETY, PERKIN TRANSACTIONS 1 1983 , LETCHWORTH GB Seiten 201 - 209 N.KATAGIRI ET AL. 'Synthesis of Homo-C-Nucleosides using Pyrimidinylmethylenephosphoranes.'
- JOURNAL OF PHARMACEUTICAL SCIENCES Bd. 67, Nr. 7 , 1978 Seiten 962 - 964 W.O.FOYE ET AL. 'Pyridinium- and Quinolinium-2-dithioecetic Acid Zwitterions: Antiradiation an Anticancer Activities.'
- SYNTHESIS. Nr. 1 , Januar 1985 , STUTTGART DE Seiten 80 - 81 J.HUNG ET AL. 'Fluoride- Assisted Nucleophilic Substitution of 6-Alkyl-5-bromouracils with Nitrogen-containing Heterocycles.'

## Beschreibung

Die Erfindung betrifft ein Verfahren zur Herstellung von C-Nukleosiden und C-Nukleosid-Analoga, neue C-Nukleoside und C-Nukleosid-Analoga und ihre Verwendung.
Die neuen C-Nukleoside und C-Nukleosidanaloga konnten bisher nicht synthetisiert werden.

6-(D-Ribofuranosylmethyl)-pyrimidine sind bekannt aus J. Org. Chem. 43, 2925 (1978), 47, 5115 (1982), 51, 1058 (1986), Liebigs Annalen 6, 957 (1986), J. Chem. Soc. Perkin I 201 (1983).

Für die Therapie von Virus - und von Krebserkrankungen ist eine Reihe von Nukleosiden synthetisiert und getestet worden. Mehrere davon werden heute in der medizinischen Praxis angewendet, u.a. Acyclovir (Schaeffer et al, Nature 1978, 272, 583-85)und 3'-Azidothymidin (Broder et al, Proc. Natl. Acad. Sci., USA, 1985,82,7096). Bei anderen steht der Einsatz als Therapeutikum kurz bevor, u.a. bei 5-(2-Bromvinyl)-2'-desoxyuridin und 3'-Fluorthymidin.

Ein Nachteil aller dieser Verbindungen liegt in ihrer Instabilität. Die N-C-Bindung zwischen dem Heterocyclus und dem Zuckerteil wird relativ leicht hydrolytisch oder enzymatisch gespalten, wonach unwirksame Abbauprodukte entstehen (York, J.; J org. Chem. 1981 (46), 2171; Mansuri, M., J. Med. Chem. 1989 (32), 461). Bei Acyclovir muß dem Patienten z.B. die 10-fache Menge der therapeutisch notwendigen Dosis verabreicht werden. Beim 5-(2-Bromvinyl)-2'-desoxyuridin sind nach 6 Stunden im Körper des Patienten nur noch etwa 1% der eingesetzten Menge vorhanden. 3'-Azido- und 3'-Fluorthymidin sind zwar hochwirksam, aber für eine Dauerbehandlung eines Patienten zu toxisch.

Bei den vom Adenin abgeleiteten 2',3'-Didesoxyadenosin treten darüber hinaus Desaminierung und Nucleosid-Spaltungen ein, was den Einsatz der an sich hochwirksamen Verbindung entscheidend limitiert (Nucleosides & Nucleotides 8 (56), 659-71 (89) und J. Med. Chem. 1988, 31, 2040-48). Man hat versucht, durch Ersatz des Adenosinrestes durch Inosin eine Verbesserung herbeizuführen, da im Körper eine geringe Umwandlung in Adenosin erfolgt. Eine entscheidender Durchbruch war aber auch damit nicht zu erreichen.

Die Erfindung hat das Ziel, therapeutisch, insbesondere virostatisch und cancerostatisch wirksame Verbindungen zur Verfügung zu stellen, welche bei einer mit den bekannten Substanzen vergleichbaren Wirksamkeit eine hohe Stabilität aufweisen. Sie sollen als potentielle Therapeutika mit geringer Belastung für den Patienten geeignet sein. Ein weiteres Ziel der Erfindung besteht darin, Bausteine für neuartige Oligonukleotide bereitzustellen.

Die Zielstellung der Erfindung wird mit den neuen C-Nukleosiden der allgemeinen Formel I erreicht; siehe Zeichnungsblatt 1/4 und 2/4.

Diese Verbindungen sind bedeutend stabiler als ihre Analogen ohne -C-Brücke zwischen Nukleobase und Zuckerrest. Da der Substituent an der Methylgruppe am C6 des Pyrimidinrings beliebig variiert werden kann, wird durch die Erfindung eine große Zahl von Verbindungen I zur Verfügung gestellt, die eine selektive Hemmung an Enzymen hervorrufen, welche für die Vermehrung von Viren bedeutungsvoll sind. Besonders stabil sind die erfindungsgemäßen Verbindungen I gemäß der Unteransprüche 4 bis 11.

Die Verbindungen I sind virostatisch und/oder cancerostatisch wirksam. Ihre Wirksamkeit wurde an Knochenmark-Stammzellen getestet. Die Verbindungen zeigen eine mit den bekannten Substanzen vergleichbare, teilweise sogar höhere Hemmung im Knochenmark-Stammzell-Proliferationstest. Sie liegt z.B. bei 6-(β-D-2',3'-Didehydro-2',3'-didesoxyribofuranos-1-yl)methyl-4-amino-pyrimidin unter 10⁻⁴ molar.

Eine weitere, sehr wichtige Anwendungsmöglichkeit der erfindungsgemäßen Verbindungen I besteht in ihrer Verwendung als Bausteine für synthetische Oligonukleotide. Unter synthetischen Oligonukleotiden werden RNA- und DNA-Oligonukleotide, darunter vor allem Ribozyme (auch Ribozyme aus DNA- und RNA-Bausteinen) und Antisense-Oligonukleotide, verstanden. Vorzugsweise werden dafür die in Anspruch 5 genannten Verbindungen eingesetzt. Die Herstellung solcher Verbindungen erfolgt nach bekanntem Muster, einige typische Wege sollen nachfolgend am Beispiel des Thymidins beschrieben werden. Zum Aufbau von DNA-Oligonukleotiden wird Thymidin zunächst zum 5'-Dimethoxy-trityl-Derivat trityliert, danach erfolgt eine Umsetzung über Cyanoethyl(diisopropyl)phosphordiamidit. Das entstandene 5'-Di-methoxy-trityl-3'-cyanoethyl(diisopropyl)phosphamidit wird anschließend in an sich bekannter Weise im Oligonukleotid-Synthesizer weiter umgesetzt.
Zum Aufbau von RNA-Oligonukleotiden muß nach der Tritylierung ein Schutz der 2'-OH-Gruppe z. B. durch den Silylrest erfolgen. Der Aufbau von Ribozymen und von Antisense-Oligonukleotiden erfolgt in prinzipiell gleicher Weise.

In der Möglichkeit, neuartige Ribozyme aufzubauen, liegt ein großer Vorteil der Erfindung. Ein Problem der bisher bekannten Ribozyme liegt darin, daß sie nicht genügend stabil sind. Man hat versucht, die Stabilität zu erhöhen, u. a.
- durch Substitution der 2'-OH-Gruppe, z. B. durch F oder durch NH₂,
- durch Ersatz der Phosphatreste durch Thiophosphate und
- durch Ersatz der Phosphatreste durch Phosphonate.
In keinem Falle jedoch war die Stabilität der Ribozyme für die vorgesehenen Verwendungen ausreichend.

Mit den erfindungsgemäßen Verbindungen kann der Ribozymaufbau auf einem neuen Wege gezielt je nach vorgesehenem Verwendungszweck erfolgen. So kann eine geeignete Substition am C5 des Pyrimidinrings der Verbindungen I die Polarität unterschiedlich beeinflussen (z. B. führt eine 5-Fluorsubstitution zur Erniedrigung des pK-Wertes, was eine höhere Azidität bedeutet).

Für den Ribozymaufbau besonders geeignete Verbindungen sind 6-(β-D-Ribofuranos-1-yl)methyl-4-aminopyrimidin und 6-(β-D-Ribofuranos-l-yl)methyl-2-amino-4-hydroxy-pyrimidin und ihre in 2'-Stellung durch F, NH₂, H oder ara-OH substituierten Derivate. Diese Verbindungen können als Phosphatester, Thiophosphatester oder als Phosphonate eingesetzt werden.

Für den Aufbau von Antisense-Oligonukleotiden sind besonders geeignet
6-(β-D-2'-Desoxyribofuranos-1-yl)methyl-4-aminopyrimidin und
6-(β-D-2'-Desoxyribofuranos-l-yl)methyl-2-amino-4-hydroxypyrimidin und deren Ribose-Analoga.
Diese Verbindungen können ebenfalls als Phosphatester, Thiophosphatester oder als Phosphonate eingesetzt werden. Die hergestellten neuartigen Antisense-Oligonukleotide zeigen eine erhöhte spezifische Aktivität und eine erhöhte Bindungsfähigkeit am gewünschten Träger, wodurch sich verbesserte Anwendungsmöglichkeiten ergeben.

Die Erfindung betrifft ferner ein Verfahren zur Herstellung der Verbindungen I und zwar durch Umsetzung einer veresterten oder verätherten 1-Halogenose der allgemeinen Formel II (siehe Zeichnungsblatt 3/4) mit C-metallierten Pyrimidinen bzw. ihrer Mono-, Di- oder Trimethylsilyloxyäther oder -alkyläther der allgemeinen Formel III, deren ggf. in 2- und/oder 4-Stellung vorhandene NH₂-Gruppen geschützt sind, vorzugsweise durch eine Dimethyl-aminomethylengruppe (siehe Zeichnungsblatt 4/4), bei Temperaturen von -70°C bis +100°C, bevorzugt bei -70°C bis -50°C.
Das Verfahren läuft im einzelnen folgendermaßen ab.
Die Halogenose II wird in einem inerten Lösungsmittel wie Alkanen, Äthern oder Aromaten gelöst, auf -70°C bis 0°C abgekühlt und tropfenweise mit dem C-metallierten Pyrimidin III versetzt. Diese Metallverbindungen III werden durch Umsetzung der entsprechenden persilylierten oder anders verätherten Pyrimidin-Derivate mit metallorganischen Verbindungen z.B. Butyllithium oder Natriumamid, hergestellt. Anschließend wird das Reaktionsgemisch bevorzugt auf Raumtemperatur gebracht und gegebenenfalls zur Vervollständigung der Umsetzung kurz erwärmt. Nach Beendigung der Umsetzung, erkennbar an ausgefallenem Metallsalz, erhält man nach den üblichen chemischen Operationen das gewünschte Produkt.
Eine andere Variante der erfindungsgemäßen Umsetzung, bevorzugt beim Einsatz von verätherten Halogenosen,besteht darin, daß man die Halogenose II in einem inerten Lösungsmittel vorlegt und das C-metallierte Pyrimidin III unter ständigem Rühren zusetzt. Von wesentlicher Bedeutung für den erfindungsgemäßen Erfolg ist die absolute Wasserfreiheit.

Die Erfindung soll nachstehend durch Ausführungsbeispiele näher erläutert werden.

### Beispiel 1

### 6-(β-D-2'-Desoxyribofuranos-1-yl)methyl-uracil

2,09 g(5 Mmol) 2,4-Bis-(triisopropylsilyloxy)-6-methyl-pyrimidin werden in 10 ml abs. Hexan gelöst, auf -70°C abgekühlt und unter Argon tropfenweise mit 4 ml 1,5 molarer Butyllithium-Lösung in Hexan versetzt. Die Lösung läßt man auf 0°C erwärmen und gibt sie tropfenweise in eine zunächst auf -70°C abgekühlte, später bis auf 0°C sich erwärmende Lösung von 1,95 g (5 Mmol) 2-Desoxy-(3,5-di-toluyl)-ribosylchlorid in 20 ml abs. Toluol. Wenn die Reaktionslösung neutral ist, wird sie einrotiert und mit Natriummethylat entacyliert und an Kieselgel (Merck 60) mit Chloroform-Methanol (9/1) in α,β Anomere aufgetrennt.
F: 125°, M: 242,2 ≙ C₁₀H₁₄N₂O₅ laut MS Varian CH₇
300 MHz NMR: H' = 6,4146 ppm ≙ β

Das β-Anomere kann nach bekannten Methoden in Cytosin-, Isocytosin- u.a. Derivate umgesetzt werden, die den Purinderivaten Adenin, Inosin, Xanthin, Guanin u.a. entsprechen.

### Beispiel 2

### 6-(β-D-2'-Desoxyribofuranos-1-yl)methyl-4-hydroxypyrimidin

12,54 g (∼30 Mmol) 4-(Triisopropylsilyloxy)-6-methyl-pyrimidin (TK 196°C) werden in 30 ml abs. Toluol gelöst und bei 0°C unter Rühren mit 20 ml 1,6 m Butyllithium in Hexan tropfenweise versetzt (Schutzgas). Die Lithiumsalzlösung wird bei 0°C in eine gerührte Lösung von 10g (∼30 Mmol) 2-Desoxy-(3,5-di-toluyl)ribosylchlorid in 100 ml abs. Toluol getropft. Zur Vervollständigung der Reaktion wird danach kurz erwärmt. Die Toluollösung wird mit kaltem Wasser extrahiert, die organische Phase getrocknet (MgSO₄), das Lösungsmittel verdampft und der Rückstand mit 100 ml 1/20m NaOCH₃-Lösung entacyliert. Die Lösung wird danach mit Ionenaustauscher Dowex H+ neutralisiert, das Glykosid und unumgesetztes Pyrimidin werden nachfolgend vom Ionenaustauscher abgelöst und über eine Kieselgelsäule in α- und β-Anomere getrennt.
Sirup, M= 226,23 ≙ C₁₀H₁₄N₂O₄ Massenpeak CH₇ (Varian)

### Beispiel 3

### 6-(β-D-2',3'-Didehydro-2',3'-didesoxyribofuranos-1-yl)methyl-4-hydroxypyrimidin

226 mg(1mmol) 6-(β-D-2'-Desoxyribofuranos-1-yl)methyl-4-hydroxypyrimidin werden in bekannter Weise in 20 ml abs. Pyridin mit 300 mg Tritylchlorid und nach weiteren 20 Stdn. bei 0°C mit 0,25 ml Mesylchlorid versetzt. Nach Hydrolyse der überschüssigen Chloride in Wassereis wird der Rückstand in Chloroform aufgenommen, nacheinander in kalter verd. Schwefelsäure, Wasser und Bicarbonatlösung extrahiert.
Die Chloroformlösung wird einrotiert, der Rückstand in Acetanhydrid erwärmt, bis der Pyrimidinring vollständig acyliert ist. Eine teilweise Detritylierung stört die nachfolgende Eliminierung nicht. Der nach erneutem Einrotieren erhaltene Rückstand wird in 5 ml DMF aufgenommen und mit 5 ml Ethyldiisopropylamin unter Rückfluß gekocht, bis die Ausgangsverbindung nicht mehr im Dünnschichtchromatogramm nachweisbar ist. Nach erneutem Einrotieren werden die Schutzgruppen in bekannter Weise entfernt und der verbliebene Rückstand an Kieselgel 60 (Merck) mit Chloroform/Methanol 9/1 getrennt.
Die Titelverbindung wird durch die für En-Verbindungen typische Bromentfärbung nachgewiesen.

### Beispiel 4

### 6-(β-D-2',3'-Didehydro-2',3'-didesoxyribofuranos-1-yl)methyl-4-aminopyrimidin

50 mg der in Beispiel 3 hergestellten Verbindung werden in 2 ml Hexamethyldisilazan und 1 Tropfen DMF langsam erwärmt, bis die Substanz in Lösung geht. Überschüssiges Hexamethyldisilazan wird im Vakuum entfernt, und der Rückstand wird mit 2 ml verflüssigtem Ammoniak und katalytischen Mengen Ammoniumchlorids im Bombenrohr bei 160°C über Nacht erwärmt. Nach Verdampfen des Ammoniaks wird der Rückstand in Methanol aufgenommen und an Kieselgel 60 (Merck) mit Chloroform/Methanol 9/1 getrennt.

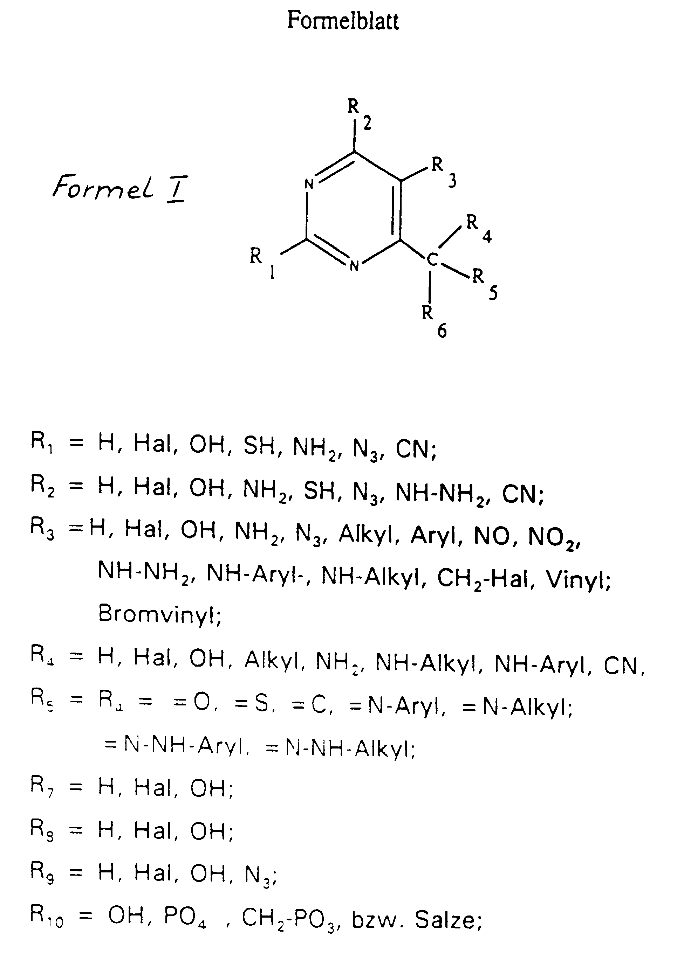

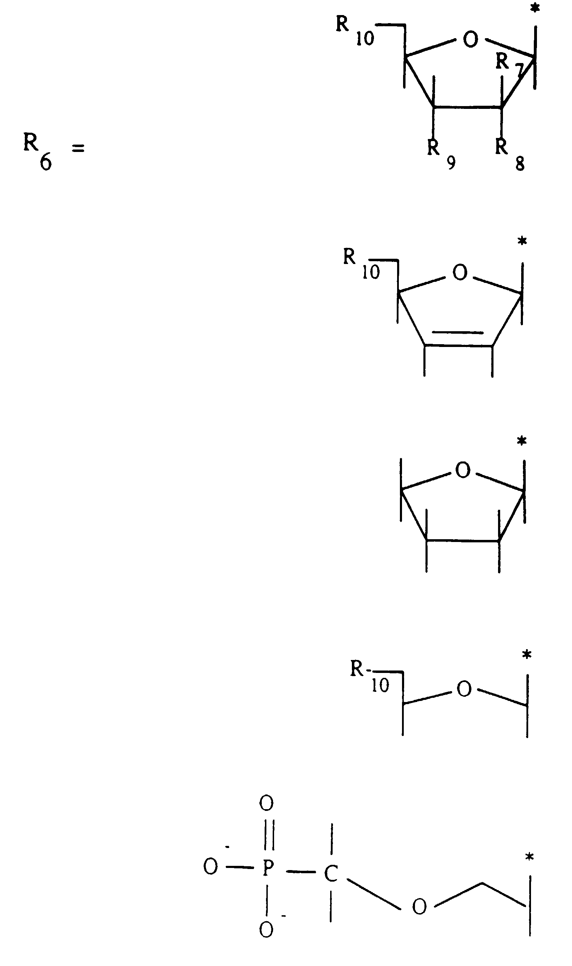

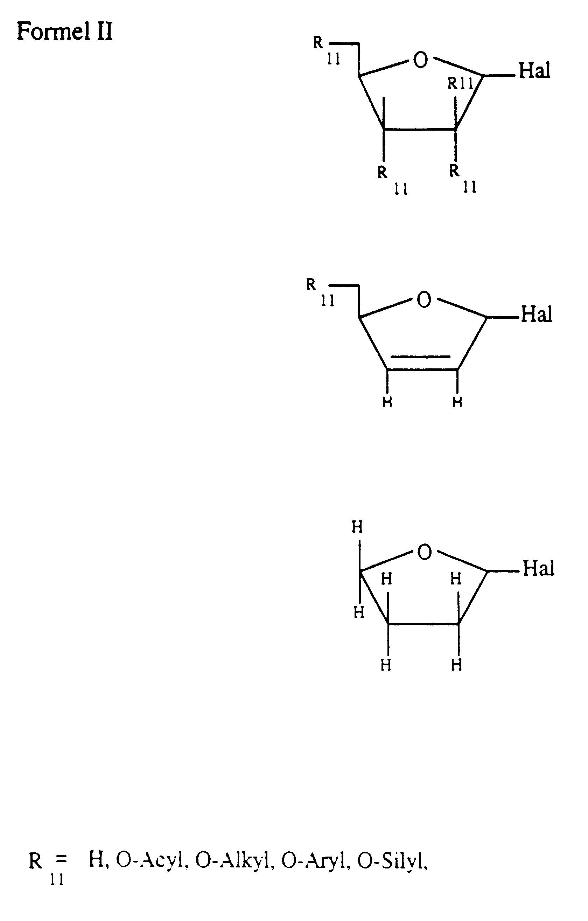

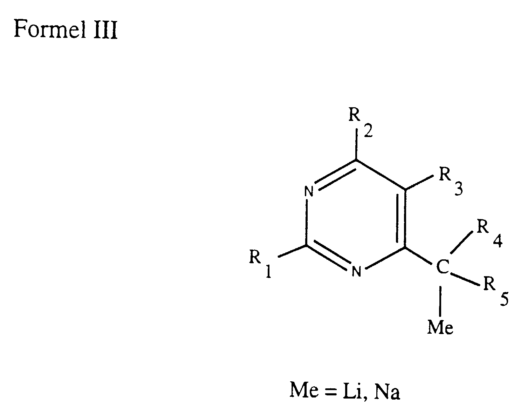

## Patentansprüche

1. Verfahren zur Herstellung von C-Nukleosiden und C-NukleosidAnaloga der allgemeinen Formel I 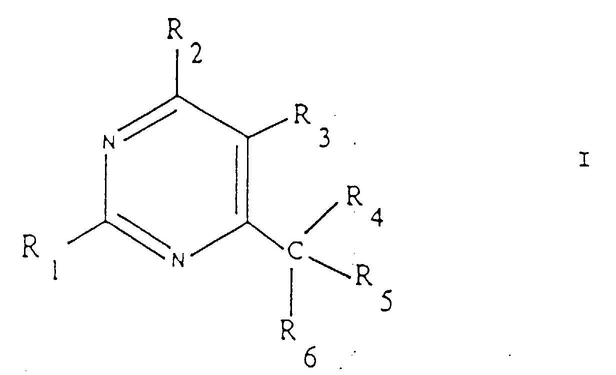 wobei
R₁ = H, Hal, OH, SH, NH₂, N₃, CN,
R₂ = H, Hal, OH, SH, NH₂, N₃, CN, NH-NH₂
R₃ = H, Hal, OH, NH₂, N₃, Alkyl, Aryl, NO, NO₂ NH-NH₂, NH-Aryl, NH-Alkyl, CH₂-Hal, Vinyl, Bromvinyl,
R₄ = H, Hal, OH, Alkyl, -NH₂, NH-Alkyl, NH-Aryl, CN
R₅ = H, Hal, OH, Alkyl, NH₂, NH-Alkyl, NH-Aryl, CN, R₄+R₅ zusammen = O, S, N-Aryl, N-Alkyl, N-NH-Aryl, N-NH-Alkyl,
R₆ = 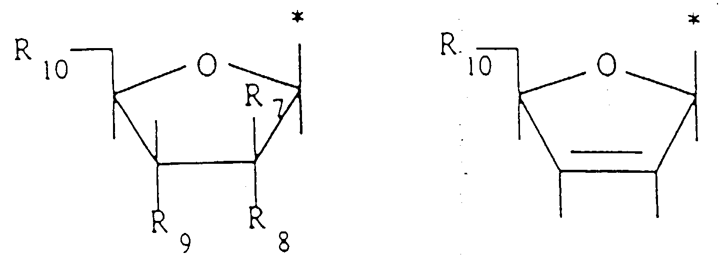 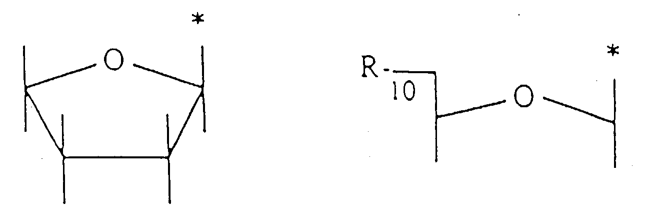 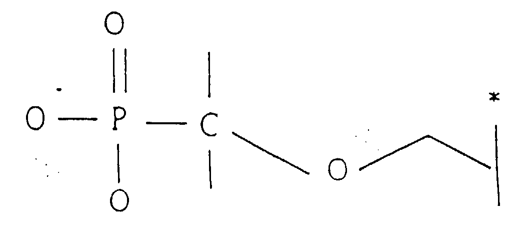 und
R₇ = H, Hal, OH,
R₈ = H, Hal, OH,
R₉ = H, Hal, OH, N₃
wobei wenn R₇ = OH, R₈ nicht OH sein kann und umgekehrt R₁₀ = OH, PO₄, CH₂-PO₃, bzw. Salze
bedeuten,
dadurch gekennzeichnet, daß man eine veresterte oder verätherte 1-Halogenose der allgemeinen Formel II 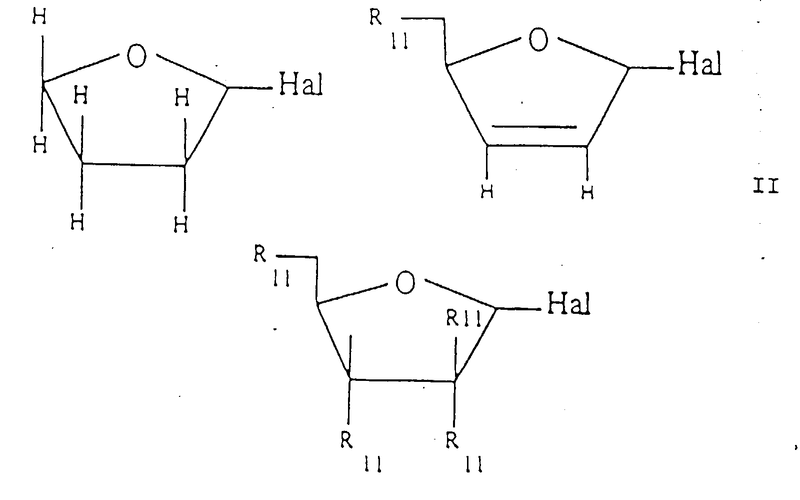 wobei R₁₁ = H, O-Acyl, O-Alkyl, O-Aryl, O-Silyl,
mit C-metallierten Pyrimidinen bzw. ihren Mono- oder Bis-, Trimethylsilyloxyäthern oder -alkyläthern der allgemeinen Formel III, deren ggf. in 2- und/oder 4-Stellung vorhandene NH₂-Gruppen geschützt sind, vorzugsweise durch eine Dimethyl-aminomethylengruppe, 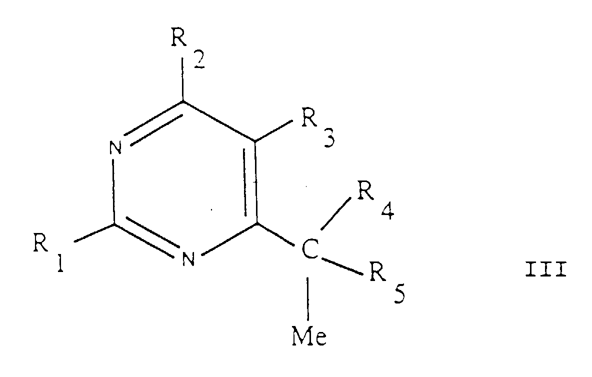 in der R₁ bis R₅ die o.g. Bedeutung hat und
Me = Li, Na ist,
bei Temperaturen von -70°C bis +100°C umsetzt, vorzugsweise bei -70°C bis -50°C, und nachfolgend gegebenenfalls mit in der Nukleosidchemie üblichen Operationen weiter umsetzt.

2. Verfahren nach Anspruch 1, dadurch gekennzeichnet, daß die Halogenose II bei -70°C bis 0°C in einem inerten Lösungsmittel, vorzugsweise in Alkanen, Äthern oder Aromaten, gelöst und tropfenweise mit dem C-metallierten Pyrimidin III im gleichen Lösungsmittel versetzt wird, das Reaktionsgemisch nachfolgend auf Raumtemperatur gebracht und gegebenenfalls zur Vervollständigung der Umsetzung kurz erwärmt wird.

3. Verfahren nach Anspruch 1 und 2, dadurch gekennzeichnet, daß man die entsprechenden persilylierten Pyrimidin-Derivate mit metallorganischen Verbindungen wie Butyllithium oder Natriumamid umsetzt und die gebildete Verbindung III im Reaktionsgemisch direkt mit der Halogenose II umsetzt.

4. 6-Methylensubstituierte Nukleoside des
4-Hydroxypyrimidins,
4-Aminopyrimidins,
2-Amino-4-hydroxypyrimidins und des
2,4-Diaminopyrimidins
und ihre 5-Halogen- und 5-Aminoderivate mit folgenden Kohlenhydratkomponenten:
2',3'-Didesoxy-ribose
3'-Azido-2',3'-didesoxy-ribose
3'-Fluor-2',3'-didesoxy-ribose
2'-Fluor-ara-2',3'-didesoxy-ribose
2',3'-Didehydro-2',3'-didesoxy-ribose.

5. 6-(β-D-2',3'-Didesoxyribofuranos-1'-yl)methyl-4-hydroxypyrimidin.

6. 6-(β-D-2',3'-Didesoxyribofuranos-1'-yl)methyl-4-aminopyrimidin.

7. 6-(β-D-2',3'-Didehydro-2',3'-didesoxy-ribofuranos-1'-yl)methyl-4-hydroxypyrimidin.

8. 6-(β-D-2',3'-Didehydro-2',3'-didesoxy-ribofuranos-1'-yl) methyl-4-aminopyrimidin.

9. 6-(β-D-2',3'-Didesoxy-3'-fluor-ribofuranos-1'-yl)methyl-4-hydroxypyrimidin.

10. 6-(β-D-2',3'-Didesoxy-3'-fluor-ribofuranos-1'-yl)methyl-4-aminopyrimidin.

11. 6-(β-D-2',3'-Didesoxy-3'-azido-ribofuranos-1'-yl)methyl-4-hydroxypyrimidin.

12. Pharmazeutische Zusammensetzung enthaltend Verbindungen I als Virostatikum.

13. Pharmazeutische Zusammensetzung enthaltend Verbindungen I als Krebstherapeutikum.

14. Verwendung der Verbindungen I als Bausteine für synthetische Oligonukleotide.

15. Verwendung nach Anspruch 14 als Bausteine für synthetische RNA-Oligonukleotide.

16. Verwendung nach Anspruch 14 als Bausteine für synthetische DNA-Nukleotide.

17. Verwendung nach Anspruch 14 und 15 zur Synthese von neuartigen Ribozymen.

18. Verwendung nach Anspruch 14-17 zur Synthese von Ribozymen aus DNA- und RNA-Bausteinen.

19. Verwendung nach Anspruch 14 und 16 bis 18 zur Synthese von neuartigen DNA-Antisense-Oligonukleotiden.

20. Verwendung nach Anspruch 14, 15, 17 und 18 zur Synthese von neuartigen RNA-Antisense-Oligonukleotiden.

## Claims

1. Process for the preparation of C-nucleosides and C-nucleoside analogues of the general formula I 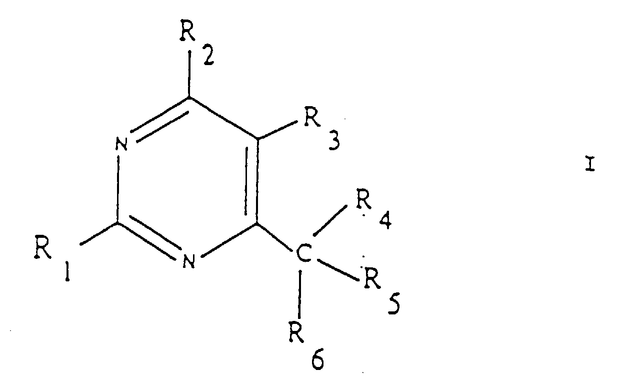 where
R₁= H, Hal, OH, SH, NH₂, N₃, CN,
R₂ = H, Hal, OH, SH, NH₂, N₃, CN, NH - NH₂
R₃ = H, Hal, OH, NH₂, N₃, alkyl, aryl, NO, NO₂ NH-NH₂, NH-aryl, NH-alkyl, CH₂-Hal, vinyl, bromovinyl,
R₄ = H, Hal, OH, alkyl, NH₂, NH-alkyl, NH-aryl, CN
R₅ = H, Hal, OH, alkyl, NH₂, NH-alkyl, NH-aryl, CN, R₄+R₅ together =0, S, N-aryl, N- alkyl, N-NH-aryl, N-NH-alkyl,
R₆ = 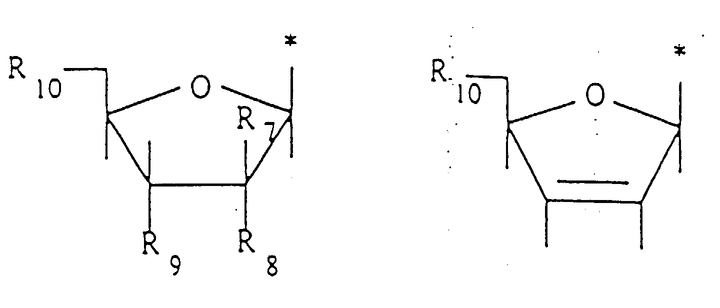 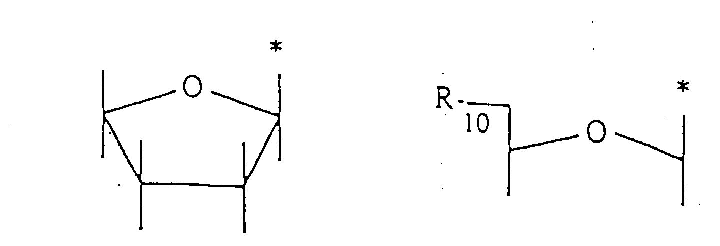 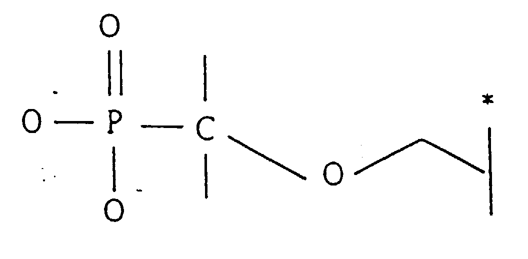 and
R₇ = H, Hal, OH,
R₈ = H, Hal, OH,
R₉ = H, Hal, OH, N₃
where if R₇ = OH, R₈ may not be OH and vice verse
R₁₀ = H, PO₄, CH₂ - PO₃ or salts,
characterized by the fact that esterified and etherified 1-halogenosis of the general formula II 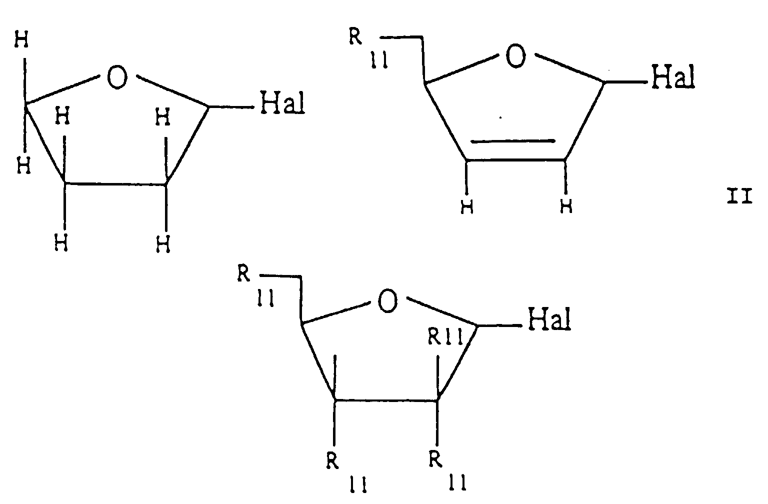 where R₂₂ = H, O-acryl, O-alkyl, O-aryl, O-silyl
is converted with C-metallized pyrimidines or their mono- or bis-, trimethylsilyloxyethers or -alkylethers of the general formula III - the NH₂ groups of which existing in the 2rd and/or 4th position being possibly protected, preferentially by a dimethylaminomethylene group 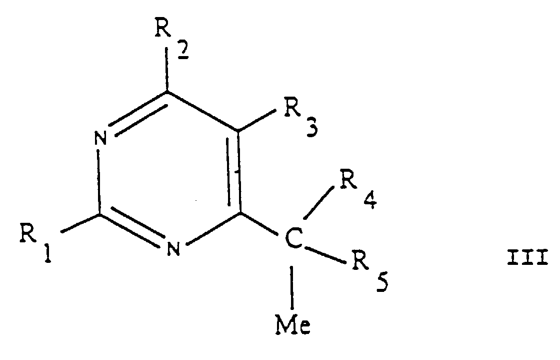 where R₁ to R₅ has the afore-mentioned meaning and
Me = Li, Na -
at temperatures between -70°C and +100°C, preferentially between -70°C and -50°C, reacting subsequently possibly in operations usual in the nucleoside chemistry.

2. Process according to claim 1, characterized by the fact that halogenosis II is dissolved at -70°C to 0°C in an inert solvent, preferentially in alkanes, ethers or aromates, and C-metallized pyrimidine III is added dropwise to the same solvent, the reaction mixture is subsequently heated to reach room temperature and, if necessary, warmed for a short time to complete the reaction.

3. Process according to claims 1 and 2, characterized by the fact that the respective persilylated pyrimidine derivatives are converted with metallo-organic compounds such as butyl lithium or sodium amide, converting compound III formed in the reaction mixture directly with halogenosis II.

4. 6-methylene substituted nucleosides of
4-hydroxypyrimidine,
4-aminopyrimidine,
2-amino-4-hydroxypyrimidine and
2,4-diaminopyrimidine
and their 5-halogen and 5-amino derivatives with the following carbohydrate components:
2',3'-didesoxy-ribosis
3'-acido-2',3'-didesoxy-ribosis
2'-fluorine-2', 3'-didesoxy-ribosis
2'-fluorine-ara- 2', 3'-didesoxy-ribosis
2',3'-didehydro- 2',3'-didesoxy-ribosis.

5. 6-(β-D-2',3'-didesoxyribofuranos 1'-yl) methyl 4-hydroxypyrimidine

6. 6-(β-D-2',3'-didesoxyribofuranos 1'-yl) methyl 4-aminopyridine

7. 6-(β-D-2',3'-didehydro-2',3'-didesoxyribofuranos-1'-yl) - methyl 4-hydroxypyrimidine

8. 6-(β-D-2',3'-didehydro-2',3'-didesoxyribofuranos-1'-yl) methyl-4-aminopyrimidine

9. 6-(β-D-2',3'-didesoxy-3'-fluorine-ribofuranos-1'-yl) methyl-4-hydroxypyrimidine

10. 6-(β-D-2',3'-didesoxy-3'-fluorine-ribofuranos-1'-yl) methyl-4-aminopyrimidine

11. 6-(β-D-2',3'-didesoxy-3'-azido-ribofuranos-1'-yl) methyl-4-hydroxypyrimidine

12. Pharmazeutical composition containing compounds I as virostatic agent

13. Pharmazeutical composition containing compounds I as cancer therapeutic agent

14. Use of compounds I as modules for synthetic oligonucleotides

15. Use according to claim 14 as modules for synthetic RNA oligonucleotides

16. Use according to claim 14 as modules for synthetic DNA oligonucleotides

17. Use according to claims 14 and 15 for the synthesis of ribozymes of a new type

18. Use according to claims 14 - 17 for the synthesis of ribozymes consisting of DNA and RNA modules

19. Use according to claims 14 and 16 up to 19 for the synthesis of DNA antisense oligonucleotides of a new type

20. Use according to claims 14, 15, 17 and 18 for the synthesis of RNA antisense oligonucleotides of a new type

## Revendications

1. Procédé pour la préparation des C-nucléosides et des analogues de formule générale I 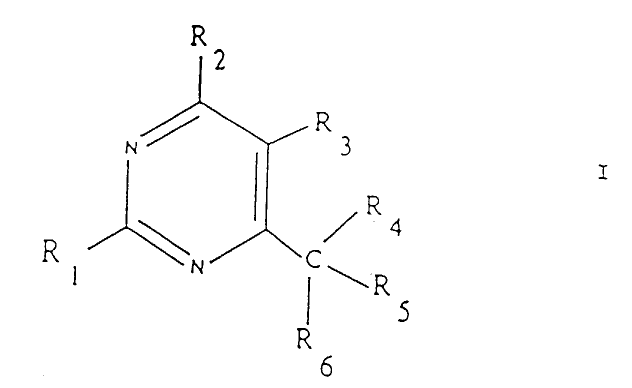 où signifient
R₁ = H, Hal, OH, SH, NH₂, N₃, CN
R₂ = H, Hal, OH, SH, NH₂, N₃, CN, NH-NH₂
R₃ = H, Hal, OH, NH₂, N₃, alkyle, aryle, NO, NO₂, NH-NH₂, NH-aryle, NH-alkyle, CH₂-Hal, vinyle, brome-vinyle
R₄ = H, Hal, OH, alkyle, NH₂, NH-alkyle, NH-aryle, CN
R₅ = H, Hal, OH, alkyle, NH₂, NH-alkyle, NH-aryle, CN, R₄ et R₅ ensemble = O, S, N-aryle, N-alkyle, N-NH-aryle, N-NH-alkyle
R₆ = 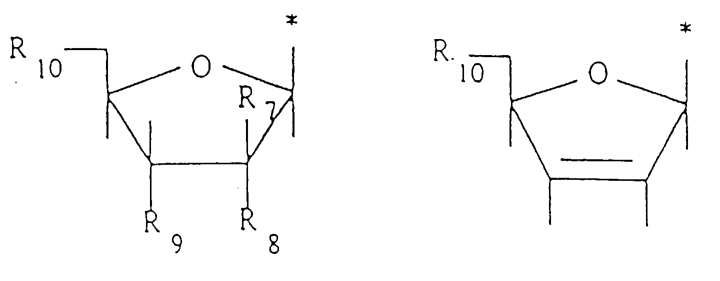 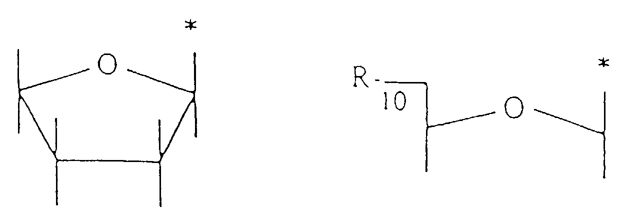 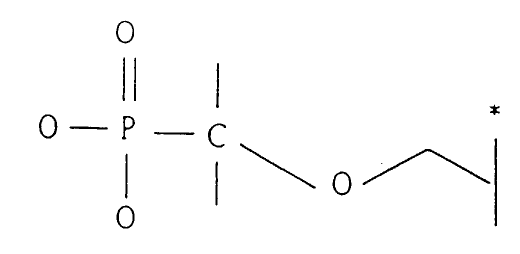 et
R₇ = H, Hal, OH
R₈ = H, Hal, OH
R₉ = H, Hal, OH, N₃
où, si R₇ = OH, R₈ ne peut être OH et vice versa
R₁₀ = OH, PO₄, CH₂-PO₃ et/ou sels,
caractérisé en ce que un 1-halogénose estérifié ou éthérifié de formule générale II 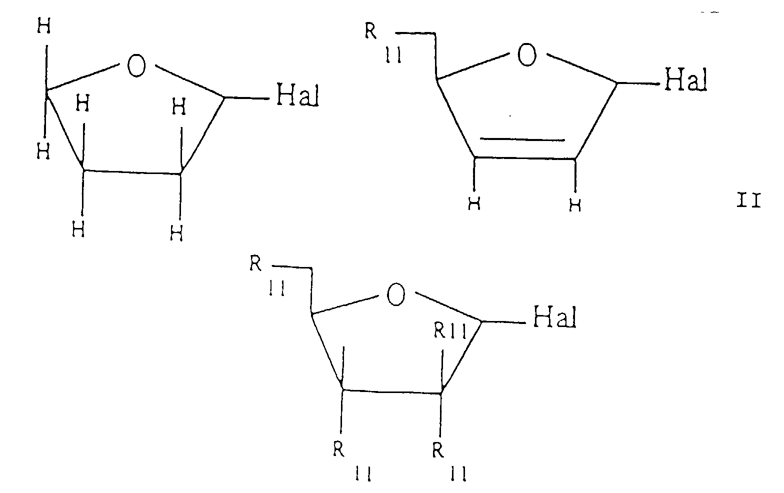 où
R₁₁ = H, O-acyle, O-alkyle, O-aryle, O-silyle
est mis en réaction avec des pyrimidines C-métallisées et/ou leurs mono- ou bis-, triméthylsilyloxyéthers ou -alkyléthers de formule générale III, dont les groupes NH₂, présents, le cas échéant, à la position 2 et/ou 4, sont protégés, de préférence par un groupe diméthylaminométhylène, 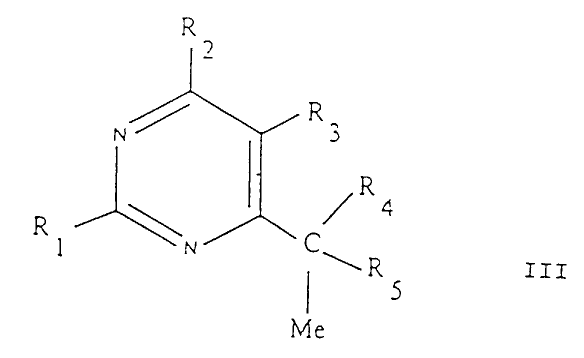 où R₁ à R₅ ont la signification susindiquée et où Me = Li, Na,
la réaction s'effectuant à des températures allant de -70 °C à +100 °C, de préférence entre -70 °C et -50 °C, et se poursuivant, le cas échéant, avec les opérations habituelles dans la chimie des nucléosides.

2. Procédé selon la revendication 1, caractérisé en ce que le halogénose II est, à une température située entre -70 °C et O °C, dissous dans un solvant inerte, de préférence des alcanes, éthers ou aromatiques, et est ajouté, goutte par goutte, avec de la pyrimidine C-métallisée III, au même solvant, que le mélange réactionnel est, par la suite, porté à température ambiante et, le cas échéant, est brièvement chauffé afin de compléter la réaction.

3. Procédé selon les revendications 1 et 2, caractérisé en ce que l'on met les dérivés de pyrimidine persilylisés en réaction avec des composés organométalliques tels que butyllithium ou amide de sodium et que le composé III, qui s'est formé, est mis en réaction avec le halogénose II directement dans le mélange réactionnel.

4. Nucléosides, substitués sur 6-méthylène, de la
4-hydroxypyrimidine,
4-aminopyrimidine,
2-amino-4-hydroxypyrimidine et de la
2,4-diaminopyrimidine
et leurs dérivés de 5-halogène et 5-aminodérivés avec les composantes d'hydrate de carbone suivantes:
2',3'-didésoxy-ribose
3'-acido-2',3'-didésoxy-ribose
3'-fluor-2',3'-didésoxy-ribose
2'-fluor-ara-2',3'-didésoxy-ribose
2',3'-didéhydro-2',3'-didésoxy-ribose.

5. 6-(β-D-2',3'-didésoxyribofuranos-1'-yl)méthyl-4-hydroxypyrimidine.

6. 6-(β-D-2',3'-didésoxyribofuranos-1'-yl)méthyl-4-aminopyrimidine.

7. 6-(β-D-2',3'-didéhydro-2',3'-didésoxy-ribofuranos-1'-yl)méthyl-4-hydroxypyrimidine.

8. 6-(β-D-2',3'-didéhydro-2',3'-didésoxy-ribofuranos-1'-yl)méthyl-4-aminopyrimidine.

9. 6-(β-D-2',3'-didésoxy-3'-fluor-ribofuranos-1'-yl) méthyl-4-hydroxypyrimidine.

10. 6-(β-D-2',3'-didésoxy-3'-fluor-ribofuranos-1'-yl) méthyl-4-aminopyrimidine.

11. 6-(β-D-2',3'-didésoxy-3'-acido-ribofuranos-1'-yl)méthyl-4-hydroxypyrimidine.

12. Constitution pharmaceutique contenant des composés I comme agent antiviral.

13. Constitution pharmaceutique contenant des composés I comme agent de traitement du cancer.

14. Utilisation des composés I comme éléments constitutifs d'oligonucléotides synthétiques.

15. Utilisation selon la revendication 14 comme éléments constitutifs d'oligonucléotides ARN synthétiques.

16. Utilisation selon la revendication 14 comme éléments constitutifs de nucléotides ADN synthétiques.

17. Utilisation selon les revendications 14 et 15 pour la synthèse de nouveaux ribozymes.

18. Utilisation selon les revendications 14 à 17 pour la synthèse de ribozymes constitués d'éléments d'ADN et d'ARN.

19. Utilisation selon les revendications 14 et 16 à 19 pour la synthèse de nouveaux oligonucléotides ADN antisense.

20. Utilisation selon les revendications 14, 15, 17 et 18 pour la synthèse de nouveaux oligonucléotides ARN antisense.
